# EUROPEAN PATENT SPECIFICATION

(11) **EP 2 808 044 B1**
(45) Date of publication and mention of the grant of the patent: **23.03.2016**
(21) Application number: 14170445.2
(22) Date of filing: 28.05.2014
(51) Int. Cl.: A61M 1/16

(54) **Dialysis machine**
Dialysemaschine
Machine de dialyse

(30) Priority: 28.05.2013 IT BO20130268
(43) Date of publication of application: 03.12.2014
(73) Proprietor: Bellco S.r.l., Mirandola (IT)
(72) Inventor: Fiorenzi, Andrea, 41018 San Cesario Sul Panaro (IT); Puviani, Fabrizio, 41038 San Felice Sul Panaro (IT); Tortola, Raffaello, 86080 Miranda (IT); Passerini, Michele, 41037 Mirandola (IT)
(74) Representative: Boggio, Luigi

(56) References cited:
- EP-A2- 0 278 100
- EP-A2- 0 665 026
- EP-A2- 2 025 355
- DE-A1- 4 139 165
- US-A1- 2011 120 946
- US-A1- 2013 049 974

## Description

The present invention relates to a dialysis machine, and in particular a dialysis machine able to use a bag containing sodium bicarbonate powder to produce a saturated bicarbonate solution to be supplied to the dialysis filter of the machine.

Containers are known that hold sodium bicarbonate powder and can be connected to the hydraulic circuit of the dialysis machine supplying a sodium bicarbonate solution to the dialysis filter so as to receive, from the hydraulic circuit, a solvent, typically water, and to supply, to the hydraulic circuit, a saturated bicarbonate solution produced by the dissolution of the bicarbonate powder in the solvent flowing through the container. These containers are disposable medical devices that, after having been used, are classified as special waste, namely waste that requires a specific disposing procedure, whose costs are substantially proportional to the volume of the material to be disposed of.

A type of container is known that holds bicarbonate powder and is made of a flexible plastic material in order to reduce manufacturing and transport costs and in order to ensure a limited size after the use. This flexible container basically consists of a plastic film bag containing bicarbonate powder and closed in a tight manner by a fitting provided with an inlet for the solvent and an outlet for the saturated bicarbonate solution. The container comprises, furthermore, a dip tube consisting of a straw, which, with one end, is connected to the outlet of the fitting and, with the other end, is dipped to the bottom of the bag so as to draw out the saturated solution without sucking in air, and a filter, which is connected to the dipped end of the straw so as to prevent bicarbonate powder from entering the hydraulic circuit of the dialysis machine. The fitting is provided with an additional mouth having a section that is larger than said inlet and outlet, so as to allow the bag to be filled with bicarbonate powder after the container has been assembled. After the filling with bicarbonate powder has ended, the additional mouth is closed.

The flexible container described above, when it is empty, after having been used, always takes up a significant space, even if it has been flattened, due to the presence of the straw and of the filter and due to the large size of the additional mouth. Furthermore, the presence of the straw and of the filter and the need to close the additional mouth after the bag has been filled are a possible source of complications for the container manufacturing process, which keep the manufacturing costs high.

Finally, despite the presence of the dip tube, a certain quantity of gas always enters the hydraulic circuit of the dialysis machine, this gas basically consisting of the carbon dioxide produced by the chemical reaction between the solvent and the bicarbonate powder and of the air contained in the particles of the bicarbonate powder. This gas is strongly undesired because dialysis liquid cannot contain gas and because the presence of gas bubbles in the hydraulic circuit can negatively affect the dynamics control function performed by the servosystems of the dialysis machine.

U.S. patent application US2013/0049974A1 relates to early detection of low bicarbonate level. In certain aspects, a method includes receiving a first signal from a conductivity detector connected to a line through which a solution of salt concentrate and fluid flows and determining a first conductivity of the solution based on the first signal. The method also includes receiving a second signal from the conductivity detector at a time after receiving the first signal, determining a second conductivity of the solution based on the second signal, and determining whether the second conductivity is less than the first conductivity by at least a threshold amount.

U.S. patent application US2011/120946A1 discloses an apparatus and a method for filling a container containing a dry powdered salt concentrate for use in dialysis with purified fluid and removing the trapped air or gases generated during the filling of the container, while maintaining the required fluid level in the container and without the need for evacuating gases from the container prior to filling.

German patent application DE4139165A1 discloses an apparatus for preparing a medicinal solution, in particular dialysis solution, from pulverulent concentrate and water comprises a water source from which a first conduit is led to a container which contains the powder and from which a second conduit into which a first measuring cell and a concentrate pump are connected leads to a mixing point. Said mixing point is located in a third conduit which leads from the water source to a consumer; downstream of the mixing point a second measuring cell is provided for monitoring the composition of the finished dialysis solution. Since the two measuring cells and lie in different conduits in which solutions of different composition are conveyed, there is no possibility whatever of confusions between concentrates of the same initial conductivity but different final conductivity after dilution with water.

European patent application EP2025355A2 discloses a container for dialysis purposes, which comprises a water inlet and a solution outlet which are located on the same side of the container, preferably within its bottom zone.

The object of the invention is to provide a dialysis machine that can work correctly despite the presence of the gases generated during the production of the bicarbonate solution, can use a bag lacking dip tubes and filters and, at the same time, can be manufactured in a straightforward and low-cost manner.

The invention is defined by the features of the independent claim.

The present invention will now be described with reference to the accompanying drawings, which show a nonlimiting embodiment thereof, wherein:
- figure 1 shows, in a plan view, the bag containing bicarbonate powder and lacking dip tubes and filters that can be used with the dialysis machine according to the present invention;
- figure 2 shows, in a perspective view, the bag of figure 1;
- figure 3 shows the lower part of the bag of figure 2, when the lower part is folded;
- figure 4 shows the flow chart of a method to manufacture the bag of figure 2;
- figure 5 shows a semifinished product obtained at an intermediate stage of the manufacturing method of figure 4;
- figure 6 shows, in a perspective view, a coupling assembly of a dialysis machine for the coupling of the connection of the bag of figure 1 to a hydraulic circuit of the machine for the distribution of a solvent and of a bicarbonate solution produced with said solvent, said machine being manufactured according to the present invention;
- figures from 7 to 10 show, in a perspective view and partially in section, the coupling assembly of figure 6 in different operating configurations; and
- figure 11 shown, in a schematic manner, the hydraulic circuit of the dialysis machine and part of the coupling assembly of figure 6, which connects the bag of figure 1 to the hydraulic circuit.

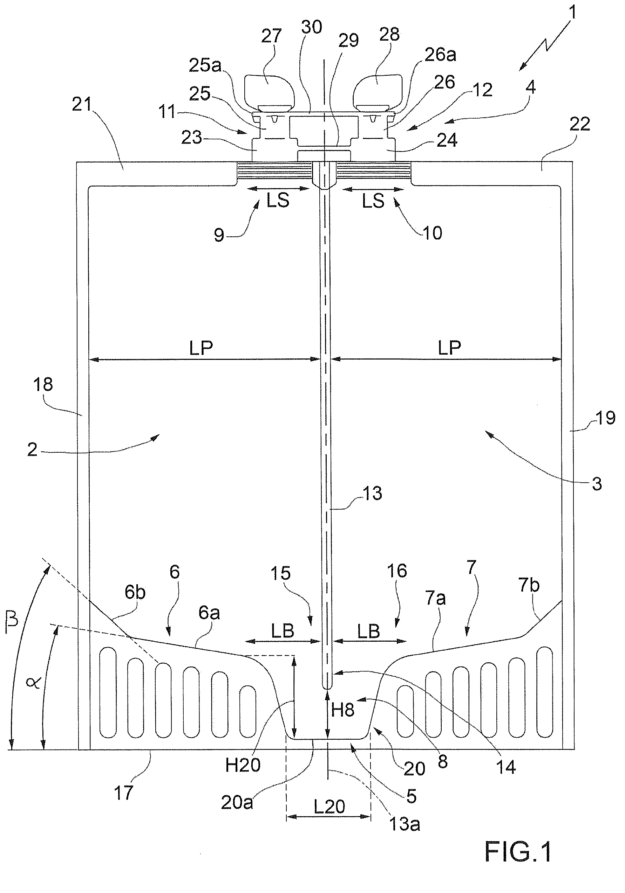

In figure 1, number 1 indicates the bag that can be used with the dialysis machine according to the present invention. The bag 1 is shown empty and flattened on the plane of the plan view. The bag 1 is divided into two pockets 2 and 3, which are suited to contain bicarbonate powder, and comprises a lower U-shaped channel 5, which establishes a communication between the bottom portions, hereinafter simply referred to as bottoms 6 and 7, of the two pockets and has a central section or portion 8 that is completely arranged under the minimum level of both bottoms 6 and 7, so that the lower channel 5 is normally full of bicarbonate powder, when, in use, the bag 1 is held with the bottoms 6 and 7 facing downwards. The pockets 2 and 3 and the lower channel 5 are manufactured as one single piece from a plastic film that is folded in two parts and welded. The plastic film is, for example, a polyamide-polyethylene (PA-PE) film. Each pocket 2, 3 is closed on the upper side, except for a respective upper opening 9, 10. The bag 1 comprises, furthermore, an upper connection 4 for the connection to the hydraulic circuit of a dialysis machine. The upper connection 4 comprises two passage elements 11 and 12, each of which is welded in a tight manner to the upper opening 9 and 10 of a respective pocket 2, 3, so as to allow a solvent, consisting for example of water, to be introduced into the bag (1) through one of the passage elements, for example element 11, and a saturated bicarbonate solution to flow out through the other passage element (12), after the solvent has spread through the bicarbonate powder from the pocket 2 to the pocket 3 flowing through the lower channel 5. The bag 1 lacks any kind of dip tube and relative filter.

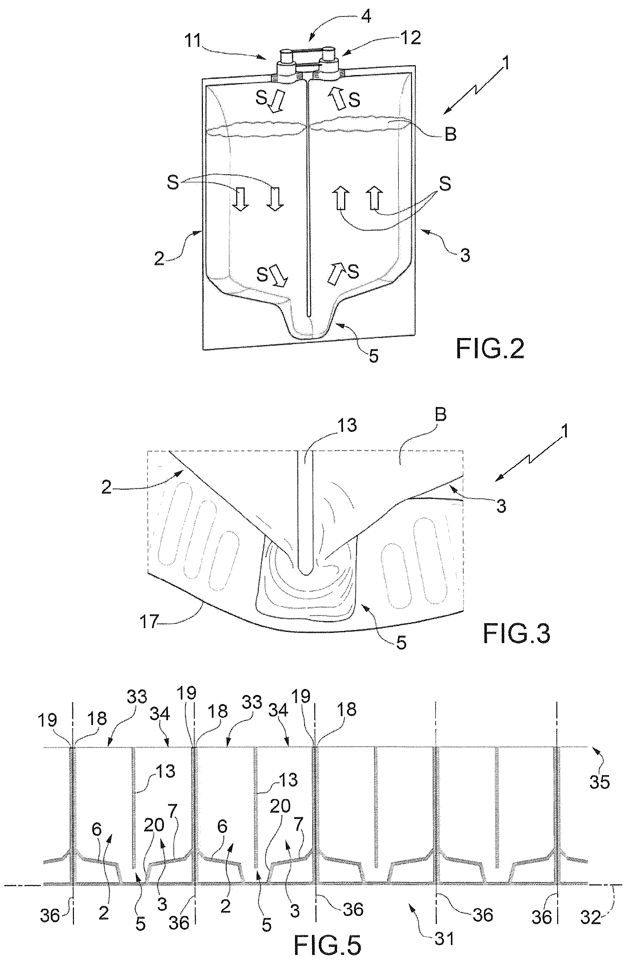

Figure 2 shows the bag 1 filled with sodium bicarbonate powder, indicated with B. The arrows indicated with S in figure 2 show the flow of the solvent, which flows in through the passage element 11, flows from the pocket 2 to the pocket 3, flows through the lower channel 5, and flows out of the passage element 12. The solvent consists, for example, of water. As the solvent flows in through the passage element 11, the saturated bicarbonate solution flows out of the other passage element 12.

With reference to figure 1, again, the lower channel 5 is delimited on the upper side by an inner side 14, which is convex, and on the lower side by an outer side 20, which is substantially concave. The two pockets 2 and 3 are symmetrical relative to a longitudinal symmetry axis 13a of the bag 1 and have the same capacity. In particular, the bag 1 has a central welded joint 13, which extends along the axis 13a so as to divide the bag 1 into the two pockets 2, 3 and so as to define, with an end of its, the inner side 14 of the lower channel 5. The bottoms 6 and 7 of the pockets 2 and 3 have, in symmetrical and close positions relative to the axis 13a, respective lower openings 15 and 16 that are contiguous with said central welded joint 13. The lower channel 5 is symmetrical relative to the axis 13a, as well, and establishes a communication between the lower openings 15 and 16. In other words, each one of the two branches of the lower channel 5 communicates with the lower opening 15, 16 of the respective pocket 2, 3.

The bottoms 6 and 7 are inclined towards the respective lower openings 15 and 16 in a specular manner relative to the axis 13a. In particular, each bottom 6, 7 comprises a first straight portion 6a, 7a, which is joined to the respective lower opening 15, 16 and is inclined, relative to a direction defined by the straight lower edge 17 of the bag 1, with an angle α ranging from 5° to 15°, and a second portion 6b, 7b, which extends from a respective lateral welded joint 18, 19 of the bag 1, which defines the outer side of the respective pocket 2, 3, to the first portion 6a, 7a and is inclined, relative to the lower edge 17, with an angle β ranging from 40° to 50°. As one can assume from figure 1, the lateral welded joints 18 ans 19 are parallel to the welded joint 13 and the lower edge 17 is perpendicular to the axis 13a.

Each lower opening 15, 16 having a width LB, which is measured perpendicular to the axis 13a and in the flattening plane of the bag 1 and is smaller than the half, and in particular smaller than one third, of the inner width LP of a pocket 2, 3, which is measured perpendicular to the axis 13a, as well. The first portion 6a, 7a of each bottom 6, 7 is joined to an end segment of the outer side 20 of the channel 5. The outer side 20 is substantially concave, in that it comprises a straight central segment defining the bottom 20a of the channel. The bottom 20a has a width L20, which is measured perpendicular to the axis 13a and is larger than the width LB, and in particular equal to approximately one third of the width LP. Each one of the two branches of the channel 5 has a length H20, which is measured parallel to the axis 13a between the lowest point of the straight portions 6a and 7a of the bottoms 6 and 7 and the bottom 20a of the channel 5 and is approximately equal to the width L20, in particular equal to approximately one third of the width LP. The central section 8 of the channel has a depth H8, which is measured along the axis 13a and is smaller that two thirds of the length H20.

The bottoms 6 and 7 and the outer side 20 of the channel 5 define a bottom line of the bag 1, which is obtained by welding, according to a corresponding shape, the lower part of the bag 1, namely the part that originates from the folding of the plastic film along the edge 17.

The upper openings 9 and 10 are in symmetrical and close positions relative to the axis 13a and are contiguous with the central welded joint 13. Each pocket 2, 3 comprises two upper flaps, which are partly welded to one another so as to form a respective upper welded joint 21, 22, that is contiguous with a respective lateral welded joint 18, 19, and, for the remaining part, define the respective opening 9, 10. Each upper opening 9, 10 has a width LS, which is measured perpendicular to the axis 13a and is substantially equal to approximately one third of the length LP.

The central welded joint 13 has a width, which is measured perpendicular to the axis 13a and ranges from 3 to 7 mm. The lateral welded joints 18 and 19 have a width that slightly larger than the one of the central welded joint 13, for example ranging from 4 to 8 mm. The upper welded joints 21 and 22 have a width, which is measured parallel to the axis 13a and ranges from 10 to 14 mm.

The passage elements 11 and 12 have a substantially circular symmetry and are identical to one another. Each passage element 11, 12 comprises a respective base portion 23, 24, whose outer peripheral surface is tightly welded to the edges of the respective upper opening 9, 10, and a respective end portion 25, 26, which is closed, on the upper side, by a respective plug 27, 28 that can be removed by hand, by breaking it, so as to permit the connection to a respective branch of a hydraulic circuit (not shown) of a dialysis machine.

The connection 4 comprises, furthermore, two flat elements 29 and 30, which are parallel to one another and substantially perpendicular to the axis 13a, so as to rigidly connect the passage elements 11 and 12 to one another, keeping a given distance. In particular, the flat element 29 connects the base portions 23 and 24, while the other flat element connects the end portions 25 and 26. The end portions 25 and 26 have respective annular edges 25a and 26a, which are coplanar and joined to the flat element 30. The connection 4 is entirely made of an injection-moulded plastic material.

In the example of figure 1, the dimensional parameters of the bag 1 are the following:
- overall height of the bag, measured along the axis 13a, excluding the connection 4, equal to 295 mm;
- overall width of the bag, measured transversely to the axis 13a, equal to 250 mm;
- width LP approximately equal to 116 mm;
- width L20 equal to 42 mm;
- height H20 equal to 42 mm;
- depth H8 equal to 25 mm;
- width LS equal to 38 mm;
- angle α approximately equal to 10°;
- angle β approximately equal to 45°;

The bag 1 of the example of figure 1 is shaped so as to hold 1300 g of sodium bicarbonate powder. For smaller quantities of bicarbonate, the bag 1 is similar to the one of figure 1, with the only difference that it has a smaller overall height.

The shape of the bag 1 is divided into the two pockets 2 and 3, which communicate at the bottom by means of the channel 5, which acts as a communicating vessel, thus eliminating the need for a dip tube and a relative filter. The absence of dip tubes and filters allows manufacturers to produce a bag that, after having been used and flattened, takes up a very small place. Furthermore, thanks to the lack of a dip tube, the number of components making up the bag 1 is significantly reduced: plastic film, bicarbonate powder, and the connection 4 for the coupling to the dialysis machine.

Thanks to the particular configuration described above, the channel 5 never completely closes during the use of the bag 1, since, when the bag 1 filled with bicarbonate powder B folds in correspondence to the channel 5, different folding lines are formed therein, which always leave a free passage between the two pockets 2 and 3, as one can assume from the example of figure 3. Furthermore, the relatively small sizes of the channel 5 allow the residual quantity of bicarbonate left in the bag at the end of a dialysis treatment to be minimized. As a matter of fact, the residual quantity of bicarbonate is substantially the one located in the trap defined by the portion of the channel 5 arranged under the imaginary line defined by the depth H8.

The absence of a dip tube permits another advantage, which is that of further simplifying the process performed to assemble and fill the bag 1.

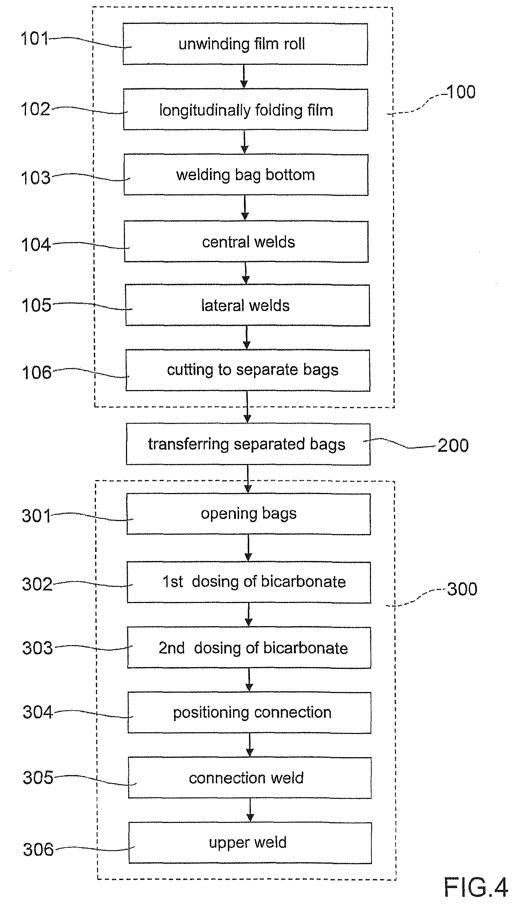

The method used to manufacture the bag 1 is implemented by an automatic apparatus of the FFS type (Form, Fill and Seal) comprising a series of operating stations controlled by a control unit. Figure 4 shows the sequence of the main processing steps performed by the manufacturing apparatus. Each one of the steps shown can be carried out by a respective operating station of the manufacturing apparatus. Figure 5 shows a semifinished product at an intermediate stage of the method to manufacture the bag 1.

With reference to figures 4 and 5, the production of the bag starts by unwinding the plastic film from a roll (step 101). The plastic film is folded along its longitudinal middle line while it is fed (step 102). The folded plastic film is fed discontinuously by means of a system of friction rollers. The feeding pitch of the friction roller system is equal to the overall width of a bag 1.

At this point, the folded film is welded in different parts so as to form a semifinished product 31 (figure 5), which comprises a succession of open bags, each of which consists of a respective pair of pockets (2, 3) and of the relative channel 5 of the bag 1, the pockets 2 and 3 being arranged transverse to the folding line 32 and being partially welded so as to be open in correspondence to respective upper sides 33 and 34, which are aligned along a side 35 of the folded film that is opposite to the side defined by the folding line 32.

In particular, on the side of the folding line 32, a series of welds having a predefined profile are performed in order to form the bottom of the bags (step 103), namely to form the bottom line of each bag 1 defined by the bottoms 6 and 7 of the pockets 2 and 3 and by the outer side 20 of the channel 5. Then, one needs to perform a series of straight welds transverse to the folding line 32, each of which corresponds to the central weld 13 of a respective bag 1 (step 104), and a further series of straight welds transverse to the folding line 32, which are alternated, in the direction of the folding line 32, with the welds 13 and are parallel thereto and each correspond to the joining of the lateral welds 18 and 19 of two bags 1 that are contiguous with one another (105).

Each weld 13, each pair of welds 18 and 19 as well as each shaped weld of the bottom of the bag 1 are performed one at a time in correspondence to relative welding stations. Each welding step is performed by means of a respective group of plates heated by electrical resistors and having the shape of the welds to be performed. Furthermore, each welding step is followed by a respective weld cooling step.

Finally, the semifinished product 31is cut along cutting lines 36 (figure 5) coinciding with the middle lines of the transverse welds 18, 19, so as to separate the open bags from one another (step) 106, namely so as to separate the pairs of pockets 2, 3 from one another in such a way that they are laterally defined by the respective lateral welded joints 18 and 19.

Steps 101-106, which are indicated as a whole with number 100 in figure 4, are carried out in a series of stations arranged in a line and run through by a linear conveying system which feeds, at first, the folded plastic film, then the semifinished product and in the end the separated open bags. The following processing steps, which are indicated as a whole with number 300, are carried out in a series of stations arranged around a carousel conveyor and substantially deal with the filling of the pockets 2 and 3 with the bicarbonate powder and with the closing thereof with the insertion of the relative connection 4 for the connection to the dialysis machine. The separated open bags are transferred, on by one, from the linear conveying system to the carousel conveyor by means of a mechanical hand (step 200).

In particular, after having been placed on the carousel conveyor, the pockets 2 and 3 are opened starting from the upper sides 33 and 34, namely the flaps of the upper side 33, 34 of each pocket 2, 3 are mutually moved apart so as to allow the open bag to be filled (step 301), and then they are simultaneously filled with bicarbonate powder, thus introducing the latter through the upper sides 33 and 34 by means of two subsequent dosing steps (steps 302 and 303). For each open bag that has been filled, the connection 4 is positioned with the respective passage elements 11 and 12 in the two upper sides 33 and 34 (step 304), the connection 4 is welded to part of the flaps of the upper sides 33 and 34 (step 305) and the remaining part of the flaps of the upper sided 33 and 34 is welded so as to completely close the pockets (step 306), namely by forming the welded joints 21 and 22 shown in figure 1, thus obtaining the finished bag 1.

The bag 1 obtained with the manufacturing method described above can be used with a dialysis machine, which, in the figures from 6 to 11, is indicated as a whole with number 37.

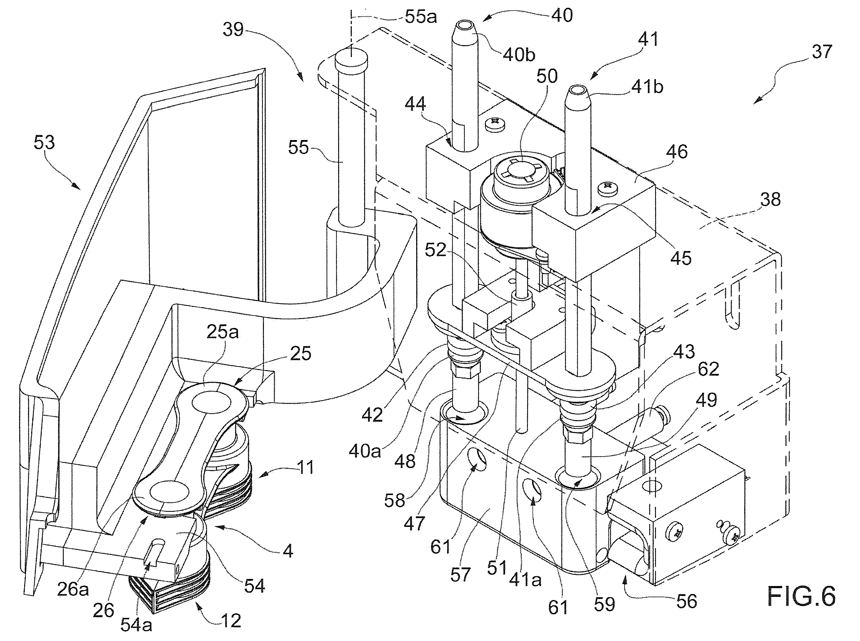

With reference to figure 6, according to the present invention, the dialysis machine 37 comprises a frame 38 and a coupling assembly 39, which is mounted on the frame 38 so as to receive the connection 4 of the bag 1 and couple the connection 4 to a hydraulic circuit (not shown) of the machine 37, said circuit being suited to feed a solvent to the bag 1 and to draw, from the bag 1, a sodium bicarbonate solution obtained from the dissolution, inside the bag 1, of the bicarbonate powder in the solvent. For the sake of simplicity, figure 6 only shows the connection 4 of the bag 1.

The coupling assembly 39 comprises two ducts 40 and 41, each of which comprises a respective coupling end 40a, 41a, which is provided with at least one sealing ring 42, 43 so as to engage, in a tight and removable manner, the end portion 25, 26 of a respective passage element 11, 12 of the connection 4. The other ends 40b and 41b of the ducts are permanently connected to the hydraulic circuit. The ducts 40 and 41 can slide in two respective vertical circular guides 44 and 45, obtained in a sub-frame 46 fixed to the frame 38, so as to be able to vertically move between a raised waiting position, which is the one shown on figure 6, and a lowered operating position. The coupling ends 40a and 41a are rigidly joined by means of a horizontal bracket 47, so that the distance between the ducts 40 and 41 is equal to the distance between the passage elements 11 and 12 of the bag 1.

The two ducts 40 and 41 comprise two respective filters 48 and 49 so that, during the coupling to the respective passage elements 11 and 12, bicarbonate powder available inside the bag 1 is prevented from entering the hydraulic circuit. In particular, each filter 48, 49 is partially fitted into the coupling end 40a, 41a of the respective duct 40, 41 in such a way that a prevailing portion of the filter 48 and 49 projects from the coupling end 40a, 41a so as to be able to penetrate the respective passage element 11, 12, when the latter is engaged by the coupling end 40a, 41a of the relative duct 40, 41.

The coupling assembly 39 comprises a motor 50, which is mounted on the sub-frame 46 and whose drive shaft ends with a worm screw 51, and a threaded element 52, which is fixed to the bracket 47 and coupled to the worm screw 51 so as to allow the ducts 40, 41 to move between the raised position and the lowered position.

The coupling assembly 39 comprises, furthermore, a cover 53, which is hinged to the frame 38 and is provided with a support 54, which is suited to receive the connection 4 of the bag 1. The hinge 55 connecting the cover 53 to the frame 38 has a substantially vertical axis 55a. The support 54 consists of a U-shaped plate, which remains horizontal during the rotation of the cover 53 relative to the axis 55a and is suited to receive the end portions 25 and 26 of the passage elements 11 and 12 of the connection 4, so that the annular edges 25a and 26a rest against the edge of the plate. The cover 53 can be manually moved between an open position, which is the one shown in figure 6 and in which the bag 1 can be loaded by inserting the connection 4 into the support 54, and a closed position, in which the passage elements 11 and 12 are in position, coaxial with the respective ducts 40 and 41, and the panel of the cover 53 covers the ducts 40 and 41 on the outside. The coupling assembly 39 comprises a hooking means 56, which is suited to be coupled, in a releasable manner, to a recess 54a obtained in the support 54, so as to hold the cover 53 in the closed position.

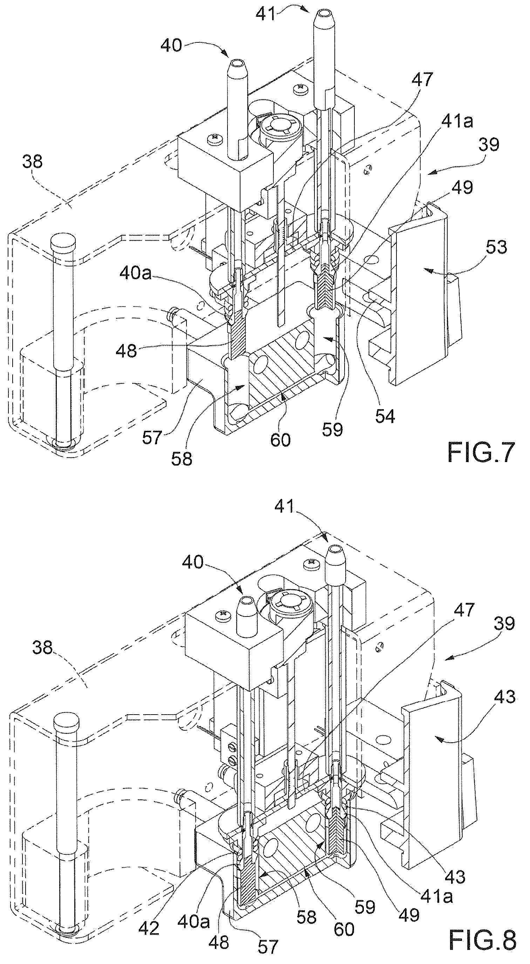

The dialysis machine 37 comprises a solid body 57, which has two vertical channels 58 and 59, having a distance that is equal to the one of the ducts 40 and 41, and has an inner duct 60 (figures 7 and 8), which establishes a mutual communication between the bottoms of the two channels 58 and 59. The body 57 has two holes 61, which are suited to be engaged, in a sliding manner, by two respective horizontal pins 62 fixed to the frame 38, so as to allow the body to horizontally move between an operating position, which is the one shown in figure 6 and in which each channel 58, 59 is coaxial to a respective duct 40, 41, and a retracted position, in which the body 57 leaves room for the connection 4. The body 57 is normally held in the operating position by an elastic element (not shown). The channels 58, 59 and 60 define a by-pass path, which is suited to close the hydraulic circuit of the dialysis machine 37, so as to allow the circuit itself to be washed.

Figure 7 shows, in a perspective view partially in section along the longitudinal axes of the ducts 40 and 41, the coupling assembly 39 in a by-pass path loading configuration, in which the channels 40 and 41 are in the raised waiting position, the body 57 is in the operating position, the bag is not inserted into the support 54, and the cover 53 is in the closed position. The U shape of the support 54, which is not completely visible in figure 7, has a width that is such as to embrace, with precision but without interference, the body 57, when the cover 53 is in the closed position.

Figure 8 shows, in the same view as figure 7, the coupling assembly 39 in a by-pass path sealing configuration, which differs from the configuration of figure 7 due to the fact that the ducts 40 and 41 are in the lowered operating position, with the coupling ends 40a and 41a engaging the respective channels 58 and 59, so that the filters 48 and 49 penetrate the channels 58 and 59 up to the level of the channel 60. The sealing rings 42 and 43 ensure a tight sealing between the outer surface of the coupling ends 40a and 41a and the inner surface of the channels 58 and 59. The washing of the hydraulic circuit is performed when the coupling assembly 39 is in the by-pass path sealing configuration.

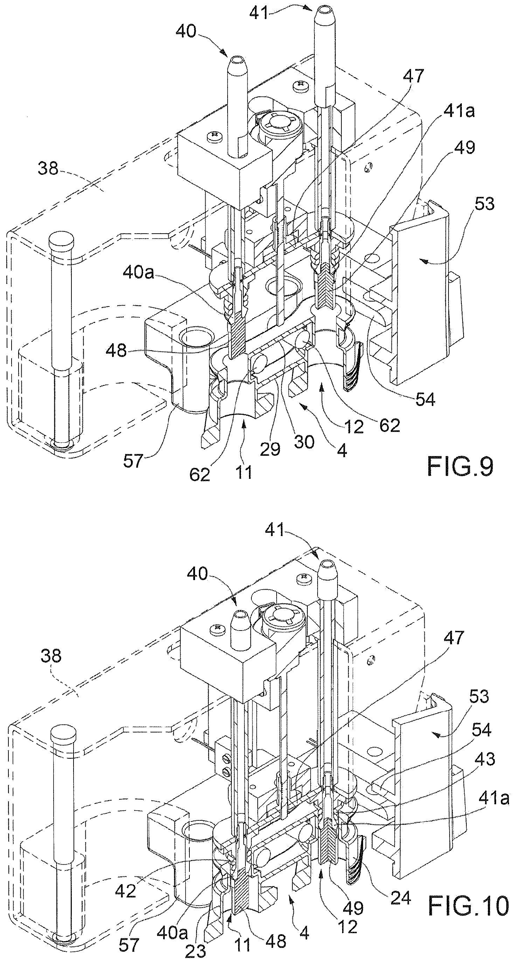

Figure 9 shows, in the same view as figure 7, the coupling assembly 39 in a bag 1 loading configuration, which differs from the configuration of figure 7 due to the fact that the connection 4 of the bag 1 is inserted into the support 54 and the cover 53, in the closed position, presses the connection 4 against the body 57 so as to hold the latter in the retracted position. The body 57, in the retracted position, leaves the pins 62 uncovered, which place themselves between the flat elements 29 and 30.

Figure 10 shows, in the same view as figure 7, the coupling assembly 39 in a bag 1 sealing configuration, which differs from the configuration of figure 9 due to the fact that the ducts 40 and 41 are in the lowered operating position, with the coupling ends 40a and 41a engaging the respective passage elements 11 and 12 of the connection 4, so that the filters 48 and 49 penetrate until they reach the inside of the base portions 23 and 24 of the passage elements 11 and 12. The sealing rings 42 and 43 ensure a tight sealing. When the coupling assembly 39 is in the bag 1 sealing configuration, the hydraulic circuit feeds the solvent to the bag 1 through, for example, the duct 40, which is tightly connected to the passage element 11, and the sodium bicarbonate solution is drawn from the bag 1 through the duct 41, which is tightly connected to the passage element 12.

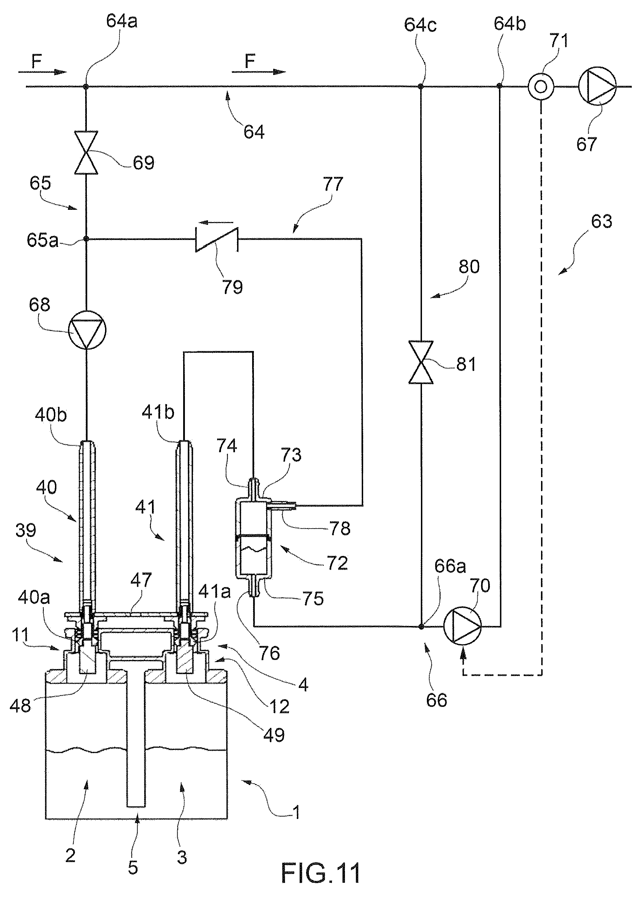

In figure 11, number 63 indicates, as a whole, the hydraulic circuit of the dialysis machine 37 for the distribution of a solvent and of a bicarbonate solution produced with said solvent. In particular, the circuit 63 is suited to feed a solvent to the bag 1 and to draw, from the bag 1, a saturated sodium bicarbonate solution produced, inside the bag 1, by the dissolution of the bicarbonate powder in the solvent. The circuit 63 comprises a main branch 64, which is flown through by the solvent according to a given circulation direction F, a solvent delivery branch 65, which connects a point 64a of the main branch 64 to the end 40b of the duct 40 of the coupling assembly 39 (figure 6), so as to the feed the solvent to the bag 1, and a solution return branch 66, which connects the end 41b of the duct 41 of the coupling assembly 39 to another point 64b of the main branch 64 arranged downstream of the point 64a relative to the circulation direction F, so as to receive the solution from the bag 1 and introduce the solution into the main branch 64. The outlet of the main branch 64, arranged downstream of the point 64b relative to the circulation direction F, supplies the bicarbonate solution to the dialysis filter (not shown).

The main body 64 comprises a pump 67, which is arranged downstream of the point 64b relative to the circulation direction F, so as to create a head that is such as to cause the solvent to circulate according to the direction F, oriented from the point 64a to the point 64 b.

The solvent delivery branch 65 comprises another pump 68 to push the solvent into the duct 40 and, thus, into the bag 1. Furthermore, the solvent delivery branch 65 comprises a pressure relief valve 69, which is arranged upstream of the pump 68 so as to limit the pressure of the solvent in the bag 1. The pressure in the bag 1 must be lower that 0.3 bar (30 kPa), otherwise there is a risk of explosion.

The solution return branch 66 comprises a pump 70 to push the solution towards the main branch 64 during the steady-state operation of the dialysis machine 37. The bicarbonate solution that reaches the solution return branch 66 mixes with the solvent coming from point 4a in the point 64b of the main branch 64. Therefore, the bicarbonate percentage dissolved in the solution at the outlet of the main branch 64, namely downstream of the point 64b relative to the circulation direction F, is linked to the bicarbonate percentage of the solution in the solution return branch 66.

The main branch 64 comprises, downstream of the point 64b relative to the circulation direction F, and in particular between the point 64b and the inlet of the pump 67, conductivity sensor 71 to provide a signal indicating the conductivity of the solution at the outlet of the main branch 64. The conductivity of the solution is liked to the percentage of bicarbonate dissolved. The pump 70 is controlled as a function of the signal provided by the conductivity sensor 71, so as to keep the percentage of bicarbonate downstream of the point 64b at a desired value.

The solvent return branch 66 comprises a dripper 72 to prevent the gases generated with the production of the solution to be introduced into the main branch 64. The gases generated during the production of the solution comprise carbon dioxide, which is a product of the reaction between the solvent and the bicarbonate powder, and air in the form of bubbles, which is released by the breaking of the particles of the bicarbonate powder when the latter is dissolved in the solvent.

The dripper 72 is arranged between the duct 41 and the pump 70. In particular, the dripper 72 consists of a container having a preferably - but not necessarily - cylindrical shape, whose ceiling 73 has an inlet 74 that is connected to the end 41b of the duct 41 and whose bottom 75 has an outlet 76 that is connected to the inlet of the pump 70. The dimensions of the dripper 72 are chosen as a function of the capacity of the bag 1. During the steady-state operation, the dripper 72 must remain substantially full of solution, so as to compensate possible emptying events caused by the air bubbles coming from the bag 1. Advantageously, the dripper 72 has a capacity ranging from 5 to 20 cm³. Higher capacity values are not recommended, because they would excessively increase the washing times of the circuit 63.

The circuit 63 comprises a gas recirculation branch 77, which connects a further outlet 78 of the dripper 72, obtained in the ceiling 73 of the dripper 72, and a point 65a of the solvent delivery branch 65, arranged between the pump 68 and the pressure relief valve 69, so as to introduce the gases generated with the production of the solution into the solvent delivery branch 65. The gas recirculation branch 77 comprises a non-return valve 79 to prevent the solvent circulating in the solvent delivery branch 65 to directly flow into the solution return branch 66 without flowing through the bag 1. The gas recirculation branch 77 brings the gases gathered in the dripper 72 back into the bag 1, so as to prevent the gases themselves from flowing through the pumps 70 and 67 and entering the main branch 64, whose outlet is connected to the dialysis filter.

The circuit 63 comprises, furthermore, a further solution return branch 80, which connects a point 66a of the other solution return branch 66 to a point 64c of the main branch 64 arranged upstream of the point 64b and comprises an on/off valve 81, which is controlled by the control unit (not shown) of the dialysis machine 37 so as to define a by-pass path for the solution that allows the initial filling and the final emptying of the bag 1 to be quickened. In particular, the point 66a is arranged between the outlet 76 of the dripper 77 and the inlet of the pump 70 and the point 64c is arranged between the point 64a and the point 64b.

In use, the on/off valve 81 is opened for an initial period so as to allow both pockets 2 and 3 of the bag 1 to be quickly filled with the solvent. A the end of the initial period, namely when the pockets 2 and 3 are presumably full of solvent, a steady-state operating period starts, during which the on/off valve 81 is closed so as to cause the solution to circulate only in the solution return branch 66, so that the flow solution directed at the main branch 64 is regulated by the pump 70. A the end of the steady-state operating period, namely when the bicarbonate of the bag 1 is close to being completely used up, a final period starts, during which the on/off valve 81 is reopened so as to allow the bag 1 to be quickly emptied.

According to a further embodiment of the present invention, which is not shown, the pump 67 is arranged upstream of the conductivity sensor 71, in particular between the point 64b and the conductivity sensor 71.

According to a further embodiment of the present invention, which is not shown, the solvent delivery branch 65 comprises an adjusted narrowing, which is arranged between the point 65a and the inlet of the pump 68, so as create a vacuum upstream of the pump 68 that is such as to draw gas or gas mixed with solution from the dripper 72.

One of the advantages of the dialysis machine 37 is that it permits the use of sodium bicarbonate bags without filters, such as bag 1, since the dialysis machine 37 already mounts the filters 48 and 49 for the bicarbonate powder on board the coupling assembly 39. Another advantage is that the gases generated with the production of sodium bicarbonate are prevented from flowing into the main branch 64 of the circuit 63, thanks to the presence of the dripper 72 on the solution return branch 66. Another advantage is a higher speed in the bag 1 filling and emptying steps, thanks to the presence of the further solvent return branch 80 provided with the on/off valve 81.

## Claims

1. A dialysis machine comprising a hydraulic circuit (63) for the distribution of a solvent and of a bicarbonate solution produced with said solvent, and coupling means (39) to couple a bag (1) containing bicarbonate powder to said circuit (63); said coupling means (39) comprising two ducts (40, 41), which can be connected to two respective passage elements (11, 12) of the bag (1); said circuit (63) comprising: a main branch (64), which is flown through by the solvent according to a circulation direction (F); a solvent delivery branch (65), which connects the main branch (64) to a first one (40) of said ducts so as to feed the solvent to the bag (1); a solution return branch (66), which connects the second duct (41) to the main branch (64), downstream of the connection to the solvent delivery branch (65) relative to the circulation direction (F), so as to receive the solution obtained from the dissolution of the bicarbonate powder in the solvent inside the bag (1) and to introduce the solution into the main branch (64); and a dripper (72), which is arranged in the solution return branch (66) so as to prevent the gases generated with production of the solution to be introduced into the main branch (64); the dialysis machine being **characterized in that** each one of said ducts (40, 41) is provided with a respective filter (48, 49) so as to prevent the bicarbonate powder from entering said circuit (63).

2. A dialysis machine according to claim 1, wherein said circuit (63) comprises a gas recirculation branch (77), which establishes a communication between the ceiling (73) of the dripper (72) and the solvent delivery branch (65).

3. A dialysis machine according to claim 2, wherein said circuit (63) comprises a non-return valve (79), which is arranged in said gas recirculation branch (77) so as to prevent the solvent circulating in said solvent delivery branch (65) to directly flow into said solution return branch (66) without flowing through the bag (1).

4. A dialysis machine according to any of the claims from 1 to 3, wherein each one of said ducts (40, 41) comprises a respective coupling end (40a, 41a), which is provided with tight sealing means (42, 43) for a tight coupling to the relative passage element (11, 12) of the bag (1); said filter (48, 49) being partially inserted into the coupling end (40a, 41a) of the respective duct (40, 41), so that an outer portion of the filter (48, 49) can penetrate the passage element (11, 12) when the latter is engaged by the coupling end (40a, 41a) of the relative duct (40, 41).

5. A dialysis machine according to any of the claims from 1 to 4, wherein said circuit (63) comprises a first pump (68), which is arranged in the solvent delivery branch (65) so as to push the solvent into said first duct (40).

6. A dialysis machine according to any of the claims from 1 to 5, wherein said circuit (63) comprises a pressure relief valve (69), which is arranged in said solvent delivery branch (65) so as to limit the pressure inside the bag (1).

7. A dialysis machine according to any of the claims from 1 to 6, wherein said circuit (63) comprises a second pump (70), which is arranged in said solution return branch (66) so as to push the solvent towards said main branch (64) during the steady-state operation of the machine.

8. A dialysis machine according to claims 1 and 7, wherein said second pump (70) is arranged downstream of said dripper (72).

9. A dialysis machine according to claim 7 or 8, wherein said circuit (63) comprises a conductivity sensor (71), which is arranged in said main branch (64), downstream of the connection to said solution return branch (66) relative to the circulation direction (F), so as to provide a signal indicating the conductivity of the solution in the main branch (64); said second pump (70) being controlled as a function of the signal provided by the conductivity sensor (71).

10. A dialysis machine according to any of the claims from 1 to 9, wherein said circuit (63) comprises a further solution return branch (80), which connects a point (66a) of the other solution return branch (66) to a point (64c) of said main branch (64) upstream of the connection to the other solution return branch (66) relative to said circulation direction (F), and an on/off valve (81), which is arranged in said further solution return branch (80) so as to quicken the initial filling and the final emptying of the bag (1).

11. A dialysis machine according to claims 1 and 10, wherein said point of the other solution return branch (66) is arranged downstream of said dripper (72).

12. A dialysis machine according to any of the claims from 1 to 11, wherein said bag comprises a connection (4) comprising said passage elements (11, 12), which are rigidly connected to one another and communicate with the inside of the bag (1); said coupling means (39) being suited to receive said connection (4) so as to couple it to said circuit (63) in a releasable manner.

13. A dialysis machine according to any of the claims from 1 to 12, wherein said bag (1) lacks any kind of dip tube and/or filter.

## Patentansprüche

1. Eine Dialysemaschine die Folgendes aufweist:
einen Hydraulikkreis (63) für die Verteilung eines Lösungsmittels und einer Bicarbonatlösung erzeugt mit dem Lösungsmittel, und Kupplungsmittel (39) zum anschließen eines Beutels (1) der Bicarbontapulver für den Kreis (63) enthält;
wobei die Kupplungsmittel (39) zwei Leitungen (40, 41) aufweisen, die mit zwei entsprechenden Durchlasselementen (11, 12) der Beutel (1) verbunden werden können;
wobei der Hydraulikkreis (63) Folgendes aufweist:
einen Hauptzweig (64) der durch das Lösungsmittel entsprechend der Rotationsrichtung (F) durchströmt wird;
einen Lösungsmittellieferzweig (65), der den Hauptzweig (64) mit einer ersten (40) der erwähnten Leitungen verbindet, um das Lösungsmittel in den Beutel (1) einzuspeisen;
einen Lösungsrücklaufzweig (66) der die zweite Leitung (41) mit dem Hauptzweig (64) verbindet und zwar relativ zur Zirkulationsrichtung (F) stromabwärts gegenüber der Verbindung mit dem Lösungsmittellieferzweig (65), um so Lösung erhalten durch die Auflösung des Bicarbonatpulvers in dem Lösungsmittel innerhalb des Beutels (1) zu empfangen und die Lösung in den Hauptzweig (64) einzuführen; und
einen Tropfenformer (72) der in dem Lösungsrückführzweig (66) derart angeordnet ist, um zu Verhindern das erzeugte Gase bei der Produktion der Lösung in den Hauptzweig (64) eingeführt werden;
wobei die Dialysemaschine **dadurch gekennzeichnet ist, dass** jede der erwähnten Leitungen (40, 41) mit einem entsprechenden Filter (48, 49) versehen ist, um so zu Verhindern, dass Bicarbonatpulver in den erwähnten Hydraulikkreis (63) eintreten kann.

2. Eine Dialysemaschine nach Anspruch 1, wobei der erwähnte Kreis (63) einen Gasrezirkulationszweig (77) aufweist, der eine Verbindung vorsieht zwischen der Decke (73) des Tropfenformers (72) und dem Lösungsmittellieferzweig (65).

3. Eine Dialysemaschine nach Anspruch 2, wobei der erwähnte Kreis (63) ein Rücklaufsperrventil (79) aufweist welches in dem Gasrezirkulationszweig (77) angeordnet ist, um so die Lösungsmittelzirkulation in dem erwähnten Lösungsmittellieferzweig (65) zu hindern direkt in den erwähnten Lösungsrücklaufzweig (66) zu fließen und zwar ohne durch den Beutel (1) zu fließen.

4. Eine Dialysemaschine nach einem der Ansprüche 1 bis 3, wobei jede Leitung der erwähnten Leitungen (40, 41) ein entsprechendes Kupplungsende (40a, 41 a) aufweist, welches mit dichten Abdichtmitteln (42, 43) versehen ist, um eine dichte Kupplung mit dem relativen Durchlasselement (11, 12) des Beutels (1) vorzusehen; wobei besagter Filter (48, 49) in das Kupplungsende (40a, 41 a) der entsprechenden Leitung (40, 41) derart eingesetzt ist, dass ein Außenteil des Filters (48, 49) das Durchlasselement (11, 12) durchdringen kann, wenn letzteres in Eingriff mit dem Kupplungsende (40a, 41 a) der entsprechenden relativen Leitung (40, 41) steht.

5. Eine Dialysemaschine nach einem der Ansprüche 1 bis 4, wobei der erwähnte Kreis (63) eine erste Pumpe (68) aufweist die in dem Lösungsmittellieferzweig (65) angeordnet ist, um so das Lösungsmittel in die erste Leitung (40) zu drücken.

6. Eine Dialysemaschine nach einem der Ansprüche 1 bis 5, wobei der erwähnte Kreis (63) ein Druckfreigabeventil (69) aufweist, welches in dem Lösungsmittellieferzweig (65) angeordnet ist, um so den Druck innerhalb des Beutels (1) zu begrenzen.

7. Eine Dialysemaschine nach irgendeinem der Ansprüche 1 bis 6, wobei der erwähnte Kreis (63) eine zweite Pumpe (70) aufweist, die in dem erwähnten Lösungsrückführzweig (66) angeordnet ist um so das Lösungsmittel zu dem Hauptzweig (64) während des Stetig-Zustandsbetriebs der Maschine zu drücken.

8. Eine Dialysemaschine nach einem der Ansprüche 1 und 7, wobei die erwähnte zweite Pumpe (70) stromabwärts von dem Tropfenformer (72) angeordnet ist.

9. Eine Dialysemaschine nach Anspruch 7 oder 8, wobei der Kreis (63) einen Leitfähigkeitssensor (71) aufweist, der in dem Hauptzweig (64) angeordnet ist und zwar stromabwärts gegenüber der Verbindung des Lösungsrückführzweigs (66) relativ zur Zirkulationsrichtung (F) um so ein Signal vorzusehen, welches die Leitfähigkeit der Lösung in dem Hauptzweig (64) anzeigt; wobei die zweite Pumpe (70) gesteuert wird als eine Funktion des durch den Leitfähigkeitssensor (71) gelieferten Signals.

10. Eine Dialysemaschine nach einem der Ansprüche 1 bis 9, wobei der Kreis (63) einen weitern Lösungsmittelrückführzweig (80) aufweist, der einen Punkt (66a) des anderen Lösungsrückführzweigs (66) mit einem Punkt (64c) des erwähnten Hauptzweiges (64) verbindet und zwar stromaufwärts der Verbindung mit dem anderen Lösungsrückführzweig (66) relativ zu der Zirkulationsrichtung (F), und ein Ein/Aus-Ventil (81), welches in dem erwähnten weiteren Lösungsrückführzweig (80) derart angeordnet ist, um das anfängliche Füllen und das schließliche bzw. finale Leeren des Beutels (1) zu beschleunigen.

11. Eine Dialysemaschine nach Ansprüchen 1 und 10, wobei der erwähnte Punkt des anderen Lösungsrückführzweiges (66) stromabwärts bezüglich des Tropfenformers (72) angeordnet ist.

12. Eine Dialysemaschine nach einem der Ansprüche 1 bis 11, wobei der Beutel eine Verbindung (4) aufweist, die die erwähnten Durchlasselemente (11, 12) aufweist die starr miteinander verbunden sind und mit der Innenseite des Beutels (1) in Verbindung stehen; wobei die Kupplungsmittel (39) geeignet sind, um die erwähnte Verbindung (4) aufzunehmen, um so diese mit dem erwähnten Kreis (63) in einer lösbaren Art und Weise zu kuppeln.

13. Eine Dialysemaschine nach einem der Ansprüche 1 bis 12, wobei der Beutel (1) keinerlei Tropfrohr und/oder Filter aufweist.

## Revendications

1. Machine de dialyse, comprenant un circuit hydraulique (63) pour la distribution d'un solvant et d'une solution de bicarbonate produite avec ledit solvant, et des moyens de couplage (39) pour coupler un sac (1) contenant une poudre de bicarbonate audit circuit (63) ; lesdits moyens de couplage (39) comprenant deux conduits (40, 41), qui peuvent être reliés à deux éléments de passage respectifs (11, 12) du sac (1) ; ledit circuit (63) comprenant : une branche principale (64), qui est parcourue par l'écoulement du solvant selon une direction de circulation (F) ; une branche de délivrance de solvant (65), qui relie la branche principale (64) à un premier (40) desdits conduits de façon à délivrer le solvant au sac (1) ; une branche de retour de solution (66), qui relie le deuxième conduit (41) à la branche principale (64), en aval de la liaison avec la branche de délivrance de solvant (65) par rapport à la direction de circulation (F), de façon à recevoir la solution obtenue à partir de la dissolution de la poudre de bicarbonate dans le solvant à l'intérieur du sac (1), et à introduire la solution dans la branche principale (64) ; et un dispositif de goutte à goutte (72), qui est disposé dans la branche de retour de solution (66) de façon à empêcher les gaz générés par la production de la solution d'être introduits dans la branche principale (64) ; la machine de dialyse étant **caractérisée en ce que** chacun desdits conduits (40, 41) est muni d'un filtre respectif (48, 49) de façon à empêcher la poudre de bicarbonate d'entrer dans ledit circuit (63).

2. Machine de dialyse selon la revendication 1, dans laquelle ledit circuit (63) comprend une branche de recirculation de gaz (77), qui établit une communication entre le plafond (73) du dispositif de goutte à goutte (72) et la branche de délivrance de solvant (65).

3. Machine de dialyse selon la revendication 2, dans laquelle ledit circuit (63) comprend une soupape antiretour (79), qui est disposée dans ladite branche de recirculation de gaz (77) de façon à empêcher le solvant circulant dans ladite branche de délivrance de solvant (65) de s'écouler directement dans ladite branche de retour de solution (66) sans s'écouler à travers le sac (1).

4. Machine de dialyse selon l'une quelconque des revendications 1 à 3, dans laquelle chacun desdits conduits (40, 41) comprend une extrémité de couplage respective (40a, 41a), qui est munie de moyens d'étanchéité étroite (42, 43) pour un couplage étroit à l'élément de passage respectif (11, 12) du sac (1) ; ledit filtre (48, 49) étant partiellement inséré dans l'extrémité de couplage (40a, 41a) du conduit respectif (40, 41), de telle sorte qu'une partie extérieure du filtre (48, 49) puisse pénétrer dans l'élément de passage (11, 12) lorsque ce dernier vient en prise avec l'extrémité de couplage (40a, 41a) du conduit concerné (40, 41).

5. Machine de dialyse selon l'une quelconque des revendications 1 à 4, dans laquelle ledit circuit (63) comprend une première pompe (68), qui est disposée dans la branche de délivrance de solvant (65) de façon à pousser le solvant dans ledit premier conduit (40).

6. Machine de dialyse selon l'une quelconque des revendications 1 à 5, dans laquelle ledit circuit (63) comprend une vanne de détente de pression (69), qui est disposée dans ladite branche de délivrance de solvant (65) de façon à limiter la pression à l'intérieur du sac (1).

7. Machine de dialyse selon l'une quelconque des revendications 1 à 6, dans laquelle ledit circuit (63) comprend une deuxième pompe (70), qui est disposée dans ladite branche de retour de solution (66) de façon à pousser le solvant vers ladite branche principale (64) durant le fonctionnement à l'état stable de la machine.

8. Machine de dialyse selon les revendications 1 et 7, dans laquelle ladite deuxième pompe (70) est disposée en aval dudit dispositif de goutte à goutte (72).

9. Machine de dialyse selon la revendication 7 ou 8, dans laquelle ledit circuit (63) comprend un capteur de conductivité (71), qui est disposé dans ladite branche principale (64), en aval de la liaison avec ladite branche de retour de solution (66) par rapport à la direction de circulation (F), de façon à délivrer un signal indiquant la conductivité de la solution dans la branche principale (64) ; ladite deuxième pompe (70) étant commandée en fonction du signal délivré par le capteur de conductivité (71).

10. Machine de dialyse selon l'une quelconque des revendications 1 à 9, dans laquelle ledit circuit (63) comprend une branche de retour de solution additionnelle (80), qui relie un point (66a) de l'autre branche de retour de solution (66) à un point (64c) de ladite branche principale (64) en amont de la liaison avec l'autre branche de retour de solution (66) par rapport à ladite direction de circulation (F), et une vanne d'ouverture/fermeture (81), qui est disposée dans ladite branche de retour de solution additionnelle (80) de façon à accélérer le remplissage initial et le vidage final du sac (1).

11. Machine de dialyse selon les revendications 1 et 10, dans laquelle ledit point de l'autre branche de retour de solution (66) est disposé en aval dudit dispositif de goutte à goutte (72).

12. Machine de dialyse selon l'une quelconque des revendications 1 à 11, dans laquelle ledit sac comprend une liaison (4) comprenant lesdits éléments de passage (11, 12), qui sont rigidement reliés entre eux et qui communiquent avec l'intérieur du sac (1) ; lesdits moyens de couplage (39) étant appropriés pour recevoir ladite liaison (4) de façon à coupler celle-ci audit circuit (63) d'une façon détachable.

13. Machine de dialyse selon l'une quelconque des revendications 1 à 12, dans laquelle ledit sac (1) ne comporte aucun type de filtre et/ou de tube plongeur.
